(19) Europäisches Patentamt
European Patent Office
Office européen des brevets

(11) EP 1 188 812 B1

(12) FASCICULE DE BREVET EUROPEEN

(45) Date de publication et mention de la délivrance du brevet:
**03.01.2007 Bulletin 2007/01**

(21) Numéro de dépôt: **01402262.8**

(22) Date de dépôt: **30.08.2001**

(51) Int Cl.:
***C10L 1/18*** *(2006.01)* ***C10L 1/02*** *(2006.01)*
***C10L 10/02*** *(2006.01)* ***C07D 307/12*** *(2006.01)*
***C08G 65/00*** *(2006.01)*

(54) **Compositions de carburants diesel contenant des composés oxygénés dérivés du tétrahydrofurfuryle**

Vom Tetrahydrofurfuryl abstammende, sauerstoffhaltige Verbindungen enthaltende Dieselbrennstoffzusammensetzungen

Compositions of diesel fuels containing oxygenates derived from tetrahydrofurfuryl

(84) Etats contractants désignés:
**DE ES IT NL**

(30) Priorité: **15.09.2000 FR 0011841**

(43) Date de publication de la demande:
**20.03.2002 Bulletin 2002/12**

(73) Titulaire: **Institut Français du Pétrole**
**92852 Rueil-Malmaison Cedex (FR)**

(72) Inventeurs:
- **Lacome, Thierry**
  **92380 Garches (FR)**
- **Montagne, Xavier**
  **92500 Rueil-Malmaison (FR)**
- **Delfort, Bruno**
  **75005 Paris (FR)**
- **Paille, Fabrice**
  **78520 Limay (FR)**

(56) Documents cités:
**WO-A-96/40844**
**GB-A- 1 539 287**
**US-A- 2 153 135**
**US-A- 2 599 338**
**US-A- 3 072 607**
**US-A- 3 328 334**
**US-A- 4 891 049**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 188 812 B1

## Description

L'invention concerne des compositions de carburant diesel contenant des composés oxygénés dérivés du tétrahydrofurfuryle.

L'amélioration de la qualité de l'air est aujourd'hui une priorité absolue de tous les grands pays industrialisés. Parmi les émetteurs de polluants référencés, le transport occupe une place qui demande que des mesures importantes soient prises pour en réduire la contribution. C'est ainsi que des trains de mesures réglementaires ont vu le jour depuis plusieurs années, avec de nouvelles contraintes dès 2000, notamment des spécifications sur la qualité des carburants. En effet, outre les caractéristiques classiquement spécifiées, de nouveaux règlements sur la composition chimique des carburants ont vu le jour, dans le but de limiter les précurseurs de certains polluants, tels que les particules, les composés réactifs vis-à-vis de l'ozone troposphérique ou les composés toxiques. Dans ce contexte, il est évident que toutes les démarches visant à améliorer la qualité des produits pour proposer des mélanges réduisant significativement les rejets polluants sont prometteuses.

C'est l'un des objets de l'invention de proposer l'utilisation de composés oxygénés dérivés du tétrahydrofurfuryle comme additifs ou comme bases de formulation des gazoles et conduisant à d'importantes baisses d'émissions de particules.

Les composés oxygénés mis en jeu dans les carburants diesel de l'invention répondent à la formule générale :

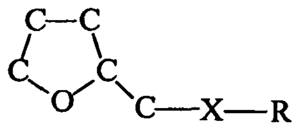

dans laquelle -X- est choisi parmi :

-O- et $-O-[CH_2-O]_n-$ avec n compris entre 1 et 20 et -R est choisi parmi :

- les groupes alkyles comprenant de 1 à 30 atomes de carbone, parmi lesquels par exemple, les groupes isopropyle, isobutyle, tertiobutyle et tertioamyle ; et
- les groupes contenant un motif tétrahydrofurfuryle, par exemple

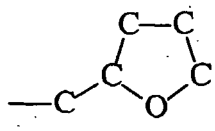

Parmi les composés que l'on préfère utiliser dans les compositions de carburant diesel de l'invention, on citera :

- l'éther tertiobutylique de tétrahydrofurfuryle (I) ; et
- l'acétal ou un polyacétal de ditétrahydrofurfuryle (II).

Leurs formules respectives sont données ci-après:

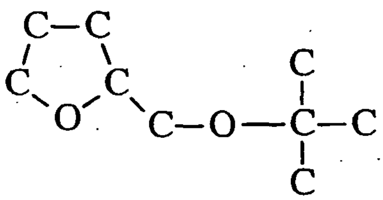

(I)

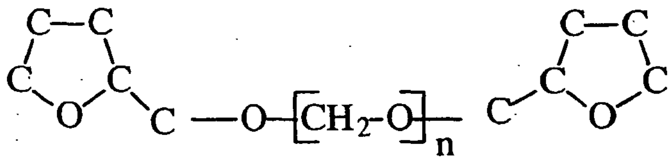 (II)

avec n compris entre 1 et 20.

On décrit ci-après les différentes voies de synthèse de ces composés préférés.

L'éther tertiobutylique de tétrahydrofurfuryle (I) peut être préparé par exemple :

- par réaction de l'alcool tétrahydrofurfurylique avec par exemple un halogénure de tertiobutyle selon un procédé d'éthérification conventionnel, faisant par exemple appel à la réaction de Williamson ; ou
- par addition de l'alcool tétrahydrofurfurylique sur de l'isobutène de préférence à l'aide d'un catalyseur acide choisi par exemple parmi l'acide sulfurique, les acides alkylbenzène sulfoniques et les résines polystyrène sulfonées, telles que la résine Amberlyst 15®. C'est la voie de synthèse qui sera décrite plus en détail dans l'Exemple 1.

L'acétal et les polyacétals de ditétrahydrofurfuryle (II) sont généralement issus de la réaction de l'alcool tétrahydrofurfurylique avec du formaldéhyde, soit sous sa forme monomère, soit sous sa forme polymère appelée paraformaldéhyde de structure $(CH_2O)_x$, soit sous sa forme trimère cyclique appelée trioxanne, de formule :

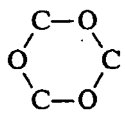

C'est cette dernière voie de synthèse qui est illustrée dans l'Exemple 2.

Cette réaction est en général catalysée par les acides comme par exemple l'acide sulfurique, les résines sulfoniques telles que la résine Amberlyst 15® ou les acides de Lewis.

Ces produits peuvent également être préparés par réaction d'échange entre l'alcool tétrahydrofurfurylique et un acétal comme par exemple le diméthoxyméthane, appelé aussi méthylal.

Dans les compositions de carburants pour moteurs diesel selon l'invention, les composés oxygénés dérivant du tétrahydrofurfuryle peuvent être utilisés en faibles concentrations, à titre d'additifs, dans des carburants diesel classiques d'origine pétrolière (distillats moyens de pétrole), contenant éventuellement des proportions variées d'autres composés oxygénés tels que les esters alkyliques dérivés d'huiles végétales. Ils peuvent également être utilisés comme additifs. La concentration en composés oxygénés de l'invention peut alors être par exemple de 0,01 à 1 %, de préférence de 0,05 à 0,2 %, en masse.

Les composés oxygénés dérivant du tétrahydrofurfuryle peuvent également être utilisés comme constituants de base du carburant diesel. Dans cette utilisation, ils peuvent représenter par exemple jusqu'à 40 % et de préférence jusqu'à 30 % en masse du carburant. Les proportions les plus usuelles de composé oxygéné dérivant du tétrahydrofurfuryle se situeront aux alentours de 10 à 15% en masse.

Dans tous les cas mentionnés ci-dessus les carburants pour moteurs diesel selon l'invention peuvent encore contenir tous autres additifs conventionnels, aux concentrations habituelles.

Selon l'invention, les carburants décrits ci-dessus peuvent alimenter tous types de moteurs diesel, à injection directe ou indirecte.

Les exemples suivants illustrent l'invention sans la limiter.

## EXEMPLES

Dans les Exemples 1 et 2, on décrit la synthèse des composés (I) et (II).

### Exemple 1 : Synthèse de l'éther tertiobutylique de tétrahydrofurfuryle (I)

La réaction mise en oeuvre est la suivante :

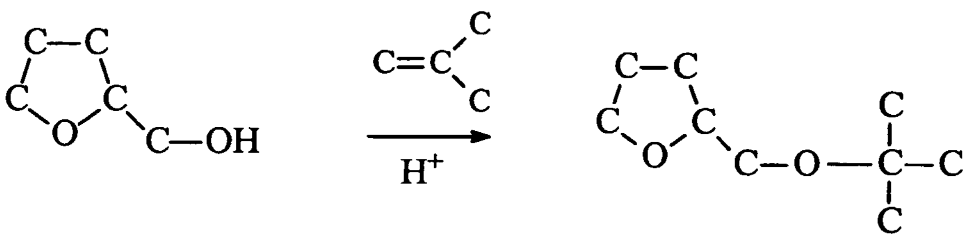


(I)

Dans un réacteur résistant à la pression, on introduit 102 g (1 mole) d'alcool tétrahydrofurfurylique, 0,3 g de résine Amberlyst 15®. On chauffe le réacteur de manière à maintenir le milieu réactionnel à 50 °C, puis on introduit de l'isobutène de manière à ajouter 68 g (1,21 mole) en 4 heures. Après retour à la température ambiante, on procède à la séparation du catalyseur par filtration, puis à l'élimination par évaporation sous pression réduite à la température ambiante de l'isobutène résiduel dissous et des éventuels sous-produits. On recueille 151 g d'un liquide incolore dont l'analyse est conforme à la structure du produit désiré.

**Exemple 2 : Synthèse de l'acétal de ditétrahydrofurfuryle (II)**

La réaction mise en oeuvre est la suivante :

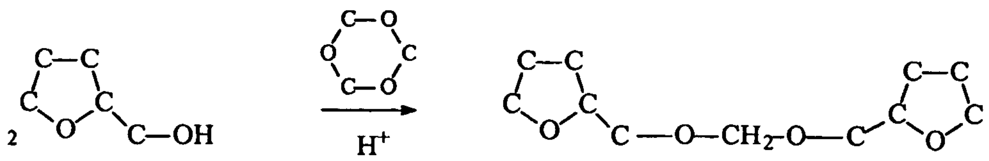


(II)

Dans un réacteur métallique fermé équipé d'un agitateur on introduit 102 g (1 mole) d'alcool tétrahydrofurfurylique, 9 g (0,1 mole) de trioxanne et 0,25 g de résine Amberlyst 15®. On porte le mélange à la température de 50 °C pendant 2 heures, puis à 90 °C pendant 1 heure. Après retour à la température ambiante, on dilue le milieu avec 250 ml de toluène, on procède à l'élimination du catalyseur par filtration, puis on évapore sous pression réduite les réactifs en excès. On obtient 55 g d'un liquide limpide dont l'analyse est conforme à la structure du produit désiré.

**Exemple 3**

On a effectué des essais ayant pour objectif d'évaluer l'impact de l'incorporation des composés oxygénés (I) et (II), pour un taux d'incorporation de 10 % en masse, correspondant à une incorporation d'oxygène allant jusqu'à 5 % en masse, sur les rejets polluants d'un moteur diesel. On a essayé, à titre de comparaison, l'alcool tétrahydrofurfurylique.
Les composés oxygénés utilisés sont notés comme suit :

- l'alcool tétrahydrofurfurylique : THFA ;
- l'éther tertiobutylique de tétrahydrofurfuryle (I) : THFA/TBE ;
- l'acétal de ditétrahydrofurfuryle (II) : THFA/acétal.

Les essais ont été menés sur un véhicule diesel équipé d'un moteur à injection directe.
Ces essais ont été effectués sur le cycle imposé par la directive 70/220/CE, modifiée par la directive 98/69/EC (cycle appelé MVEG-11s Euro 2000). Ce cycle est composé d'une phase urbaine (cycle ECE d'une longueur de 4,052 km) et d'une phase extra-urbaine (cycle EUDC d'une longueur de 6,955 km). Les résultats d'essais, présentés en g/km, sont exprimés en terme d'efficacité sur chacune des phases du cycle et sur le cycle complet.
Les essais ont été effectués à partir d'un gazole représentatif des formulations Euro 2000 (densité de l'ordre de 835 à 15 °C, teneur en soufre de l'ordre de 300 ppm, indice de cétane de l'ordre de 53, intervalle de distillation 170/366 °C).
Les résultats obtenus sont présentés dans les tableaux suivants:

| Résultats sur cycle MVEG (g/km) | | | | |
|---|---|---|---|---|
| | Gazole de référence | THFA (comp.) | THFA/TBE | THFA/acétal |
| Particules | 0,07198 | 0,0585 | 0,05915 | 0,0575 |

| Résultats sur cycle ECE (g/km) | | | | |
|---|---|---|---|---|
| | Gazole de référence | THFA (comp.) | THFA/TBE | THFA/acétat |
| Particules | 0,0603 | 0,0442 | 0,04865 | 0,04568 |

| Résultats sur cycle EUDC (g/km) | | | | |
|---|---|---|---|---|
| | Gazole de référence | THFA (comp.) | THFA/TBE | THFA/acétal |
| Particules | 0,0788 | 0,0669 | 0,0653 | 0,0584 |

L'utilisation selon l'invention de la gamme de composés oxygénés dérivant du tétrahydrofurfuryle conduit à une réduction des émissions de particules de l'ordre de 30 % sur l'ensemble des conditions testées.

## Revendications

1. Composition de carburant diesel **caractérisée en ce qu'**elle comprend au moins un composé oxygéné dérivant de tétrahydrofurfuryle et répondant à la formule générale :

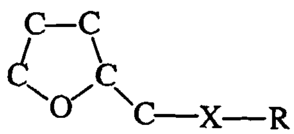

dans laquelle -X- est choisi parmi :

-O- et $-O-[CH_2-O]_n-$ avec n compris entre 1 et 20

et -R est choisi parmi :

- les groupes alkyles comprenant de 1 à 30 atomes de carbone ; et
- les groupes contenant un motif tétrahydrofurfuryle.

2. Composition de carburant diesel selon la revendication 1 **caractérisée en ce que**, dans la formule générale du composé oxygéné, les groupes alkyles comprenant de 1 à 30 atomes de carbone sont choisis parmi les groupes isopropyle, isobutyle, tertiobutyle et tertioamyle.

3. Composition de carburant diesel selon la revendication 1 ou 2 **caractérisée en ce que**, dans la formule générale du composé oxygéné, les groupes contenant un motif tétrahydrofurfuryle répondent à la formule :

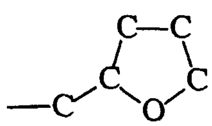

**4.** Composition de carburant diesel selon l'une des revendications 1 à 3 **caractérisée en ce qu'**elle comprend un carburant diesel classique et **en ce que** ledit composé oxygéné dérivant de tétrahydrofurfuryle, utilisé comme additif, représente de 0,01 à 1 % et de préférence de 0,05 à 0,2 % de sa masse.

**5.** Composition de carburant diesel selon l'une des revendications 1 à 3 **caractérisée en ce qu'**elle comprend un carburant diesel classique et **en ce que** ledit composé oxygéné dérivant de tétrahydrofurfuryle, utilisé comme constituant de base, représente jusqu'à 40 % et de préférence jusqu'à 30 % de sa masse.

**6.** Composition de carburant diesel selon la revendication 5 **caractérisée en ce que** la proportion du composé oxygéné dérivant de tétrahydrofurfuryle est d'environ 10 à 15% en masse.

**7.** Composition de carburant diesel selon l'une des revendications 1 à 6 **caractérisée en ce que** ledit carburant diesel classique est choisi parmi les carburants diesel d'origine pétrolière et les mélanges d'esters alkyliques dérivés d'huiles végétales.

## Claims

**1.** Composition of diesel fuel **characterised in that** it comprises at least one oxygenated compound derived from tetrahydrofurfuryl and having the general formula:

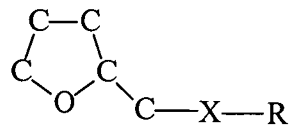

in which -X- is chosen from:
-O- and $-O-[CH_2-O]_n-$ with n comprised between 1 and 20
and -R is chosen from:

- the alkyl groups containing from 1 to 30 carbon atoms; and
- the groups containing a tetrahydrofurfuryl unit.

**2.** Diesel fuel composition according to claim 1 **characterised in that**, in the general formula of the oxygenated compound, the alkyl groups containing from 1 to 30 carbon atoms are chosen from the isopropyl, isobutyl, tertbutyl and tertamyl groups.

**3.** Diesel fuel composition according to claim 1 or 2 **characterised in that**, in the general formula of the oxygenated compound, the groups containing a tetrahydrofurfuryl unit correspond to the formula:

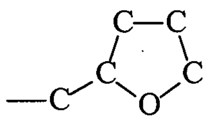

**4.** Diesel fuel composition according to one of claims 1 to 3 **characterised in that** it comprises a traditional diesel fuel and **in that** the said oxygenated compound derived from tetrahydrofurfuryl, used as an additive, represents from 0.01 to 1% and preferably from 0.05 to 0.2 % of its weight.

**5.** Diesel fuel composition according to any one of claims 1 to 3 **characterised in that** it comprises a traditional diesel fuel and **in that** the said oxygenated compound derived from tetrahydrofurfuryl, used as a basic constituent, represents up to 40 % and preferably up to 30 % of its weight.

**6.** Diesel fuel composition according to claim 5 **characterised in that** the proportion of the oxygenated compound derived from tetrahydrofurfuryl is approximately 10 to 15 % by weight.

7. Diesel fuel composition according to one of claims 1 to 6 **characterised in that** the said traditional diesel fuel is chosen from diesel fuels of petroleum origin and alkyl ester mixtures derived from vegetable oils.

## Patentansprüche

1. Dieselkraftstoffzusammensetzung, **dadurch gekennzeichnet, dass** sie mindestens eine sauerstoffhaltige Verbindung umfasst, welche sich von einer Tetrahydrofurfurylverbindung ableitet und folgender allgemeiner Formel entspricht:

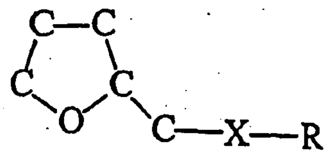

wobei X gewählt wird aus:
-O- und -O-$[CH_2\text{-}O]_n$- mit n zwischen 1 und 20
und R gewählt wird aus:

- Alkylgruppen, die 1 bis 30 Kohlenstoffatome umfassen; und
- Gruppen, die einen Tetrahydrofurfurylrest enthalten.

2. Dieselkraftstoffzusammensetzung nach Anspruch 1, **dadurch gekennzeichnet, dass** die Alkylgruppen mit 1 bis 30 Kohlenstoffatomen der allgemeinen Formel der sauerstoffhaltigen Verbindung aus den Gruppen Isopropyl, Isobutyl, tert.-Butyl und tert.-Amyl gewählt werden.

3. Dieselkraftstoffzusammensetzung nach den Ansprüchen 1 oder 2, **dadurch gekennzeichnet, dass** die Gruppen der allgemeinen Formel der sauerstoffhaltigen Verbindung, welche einen Tetrafurfurylrest enthalten, folgender Formel entsprechen:

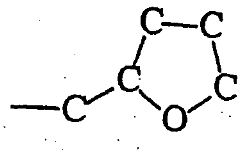

4. Dieselkraftstoffzusammensetzung nach einem der Ansprüche 1 bis 3, **dadurch gekennzeichnet, dass** sie einen herkömmlichen Dieselkraftstoff umfasst und dass ihr die sauerstoffhaltige Verbindung, die sich von einer Tetrahydrofurfurylverbindung ableitet, als Additiv in Mengen von 0,01 bis 1 Gewichtsprozent, vorzugsweise von 0,05 bis 0,2 Gewichtsprozent, zugesetzt ist.

5. Dieselkraftstoffzusammensetzung nach einem der Ansprüche 1 bis 3, **dadurch gekennzeichnet, dass** sie einen herkömmlichen Dieselkraftstoff umfasst und dass sie die sauerstoffhaltige Verbindung, die sich von einer Tetrahydrofurfurylverbindung ableitet, als Grundbestandteil in Mengen von bis 40 Gewichtsprozent, vorzugsweise von bis zu 30 Gewichtsprozent, enthält.

6. Dieselkraftstoffzusammensetzung nach Anspruch 5, **dadurch gekennzeichnet, dass** der Anteil der sauerstoffhaltigen Verbindung, die sich von einer Tetrahydrofurfurylverbindung ableitet, ungefähr zwischen 10 und 15 Gewichtsprozent liegt.

7. Dieselkraftstoffzusammensetzung nach einem der Ansprüche 1 bis 6, **dadurch gekennzeichnet, dass** der herkömmliche Dieselkraftstoff aus Dieselkraftstoffen mineralischer Herkunft und Mischungen von Alkylestern, die sich von Pflanzenölen ableiten, gewählt ist.